(19) Europäisches Patentamt
European Patent Office
Office européen des brevets

(11) EP 1 511 475 B1

(12) EUROPEAN PATENT SPECIFICATION

(45) Date of publication and mention
of the grant of the patent:
05.10.2005 Bulletin 2005/40

(51) Int Cl.⁷: **A61K 31/215**, A61P 35/02

(86) International application number:
PCT/IB2002/005565

(21) Application number: 02781696.6

(22) Date of filing: 20.12.2002

(87) International publication number:
WO 2003/101446 (11.12.2003 Gazette 2003/50)

(54) **A HERBAL MOLECULE AS POTENTIAL ANTI-LEUKEMIC DRUG**

EIN KRÄUTERMOLEKÜL ALS POTENTIELLES ANTI-LEUKÄMISCHES ARZNEIMITTEL

MOLECULE D'ORIGINE VEGETALE EN TANT QUE MEDICAMENT ANTILEUCEMIQUE POTENTIEL

(84) Designated Contracting States:
DE FR GB

(30) Priority: 31.05.2002 US 384163 P
08.07.2002 US 393750 P

(43) Date of publication of application:
09.03.2005 Bulletin 2005/10

(73) Proprietor: COUNCIL OF SCIENTIFIC AND
INDUSTRIAL RESEARCH
New Delhi 110 001 (IN)

(72) Inventors:
• BANDYOPADHYAY, Santu,
Indian Inst. Chemical Bio.
700 032 Kolkota (IN)
• PAL, Bikash Chandra, Indian Inst. Chemical Bio.
700 032 Kolkota (IN)
• BATTACHARYA, Samir,
Indian Inst. Cehmical Bio.
700 032 Kolkota (IN)
• ROY, Keshab Chandra,
Indian Inst. Chemical Bio.
700 032 Kolkota (IN)
• BANDYOPADHYAY, Gautam,
Indian Inst. Chemical Bio.
700 032 Kolkota (IN)

(74) Representative: Long, Giorgio et al
Jacobacci & Partners S.p.A.
Via Senato, 8
20121 Milano (IT)

(56) References cited:
WO-A-02/45730

• G.TIYU E.A.: "FTIR investigation of the interaction of tumor cells treated with caffeic acid and chlorogenic acid" VIBRATIONAL SPECTROSCOPY, vol. 24, no. 2, 2000, pages 225-231, XP001149867
• V.A.FUNG E.A.: "Mutagenic activity of some coffee flavor ingredients" MUTATION RESEARCH, vol. 204, no. 2, 1988, pages 219-228, XP001149534
• C. LIEN-CHAI E.A.: "Antileukemic activity of selected natural products in Taiwan" AMERICAN JOURNAL OF CHINESE MEDICINE, vol. 31, no. 1, 2003, pages 37-46, XP001149533

**Description**

**Technical Field**

This invention relates to treatment of acute and chronic myeloid leukemia (AML and CML) and also of lymphoid leukemia by Chlorogenic acid (CA) isolated from *Piper betel* leaf extract. Myeloid leukemia, both acute (AML) and chronic (CML) and lymphoid leukemia are lethal, there is no drug directing towards the destruction of the leukemic cells, and these cells poorly respond to chemotherapy which is always non-specific thus adversely affecting normal cells. Unique property of the therapy with *Piper betel* component (Chlorogenic acid) is the killing of myeloid cancer cells and lymphoid cancer cells leaving other normal cells unaffected.

**Background and prior art references:**

Myeloid leukemia is usually subdivided into two groups: Acute Myeloid Leukemia (AML) and Chronic Myeloid Leukemia (CML). AML is characterized by an increase in the number of myeloid cells in the bone marrow and an arrest in their maturation. In the United States, the annual incidence of AML is approximately 2.4 per 100,000 and it increases progressively with age, to a peak of 12.6 per 100,000 adults 65 years of age or older. The CML is a malignant clonal disorder of hematopoietic stem cells. The median age at presentation is 53 years, but it occurs at all age groups, including children. The natural history of CML is progression from a benign chronic phase to a rapidly fatal blast crisis within three to five years or even earlier. The prognosis of CML is also poor in spite of vast advancement of clinical medicine (1). CD33 represents a specific and useful marker in the process of myeloid cell differentiation (2). Recent reports suggest that engagement of CD33 by monoclonal antibody induced apoptosis leading to growth inhibition of proliferation of AML and CML cells *in vitro* (2,3). Exploiting the myeloid specific expression of CD33, humanized anti-CD33 monoclonal antibody conjugated with anti-cancer drug has been tried in AML patients with significant success (4). Similarly, lymphoid leukemia is also subdivided in two groups: acute lymphocytic leukemia (ALL) and chronic lymphocytic leukemia (CLL). Lymphoid leukemia may affect both T and B cell lineages and are prevalent in children. With the extracts from *Piper betel* leaves anti-myeloid activity was claimed earlier (Patent filed no. PCT/INOO/00118 dated December 12, 2000) and 3-o-p-Coumaryl quinic acid, an active factor for the treatment of AML and CML on 30.05.2002 US provisional patent Application No. 60/384,163 (299 / NF / 2002.)

Hence, applicant's earlier findings are in direct consonance with the present patent filing on Chlorogenic acid (CA) isolated from the fractions of the betel leaf extracts for treating acute and chronic myeloid leukemia and lymphoid leukemia. Chlorogenic acid is known to have anti-allergic activity (5). CA also inhibits hepatic and renal glucose-6-phosphatase systems (6). CA is an inhibitor of epidermal lypoxygenase activity and TPA-induced ear inflammation (7). CA also renders inhibitory effects on TPA-induced tumor promotion in mouse skin (7). Anti-HIV activity of CA has also been reported (8). Although inhibition of tumor promotion has been attributed to CA, no anti-tumor activity on established tumors including anti-leukemic activity has not been reported for CA. In the present patent application, anti-leukemic activity and the anti-tumor activity of CA are claimed for the first time.

*Piper betel* leaves have a strong pungent aromatic flavour and are widely used in India as a masticatory. Generally, mature or over mature leaves, which have ceased growing but not yet become brittle are used for chewing. The basic preparation for chewing purposes consists of betel leaf smeared with hydrated lime and catechu to which scrapings of arecanut are added; flavorings such as coconut shavings, clove, cardamom, fennel, powdered liquorice, nutmeg and also tobacco are used according to one's taste. In some places prepared *Piper betel* leaf preparation is covered with silver or gold film. As a masticatory, it is credited with many properties: it is aromatic, digestive, stimulant and carminative. Medicinally it is useful in catarrhal and pulmonary infections; it is also used for poultices. The effects of chewing of betel leaves with arecanut and other adjuncts are the excitation of the salivary glands and the irritation of the mucous membrane of the mouth. The red coloration produced is due to a pigment in the arecanut, which manifests itself under the action of alkali in lime and catechu. A mild degree of stimulation is produced, resulting in a sensation of warmth and well being, besides imparting a pleasant odour. The most important factor determining the aromatic value of the leaf is the amount and particularly the nature of the essential oil present. Betel leaves from different regions vary in smell and taste. The most pungent is the Sanchi type, while the most mild and sweet ones are from Varanasi.

The betal leaves contain essential oils, the content of oil varies from 0.7 to 2.6 per cent depending upon the varieties of leaves. The oil consists of phenols and terpens. The higher the proportion of phenol oil, the better the quality. An isomer of eugenol named chavibetol (betel phenol; 4-alkyl-2-hydroxy-Imethoxy benzene) is considered to be characteristic constituent of betel oil. Betel oil of Indian types contains as a predominant phenolic constituent and used in the treatment of various respiratory problems, either as a local application or by gargle. It has carminative properties. It exhibits in different actions on the central nervous system of mammals. The essential oil and extracts of the leaves possess activity against several Gram-positive and Gram-negative bacteria such as *Micrococcus pyogenes* var. Albus, *Bacillus subtilis* and *B. Megaterium, Diplococcus pneumoniae, Streptococcus pyogenes, Escherichia coli,*

**EP 1 511 475 B1**

*Salmonella typhosa, Vibrio comma, Shigella dysenteriae, Proteus vulgaris, Pdseudomonas solanacaerum, Sarcina lutea* and *Erwinia carotorora.* The essential oil and leaf extracts also showed anti-fungal activity against *Asperigillus niger* and *A. Oryzae, Curvularia lunata* and *Fusarium oxysporum*. The oil is found to be lethal in about 5 minutes to the protozoa, *Paramaeceum caudatum* (5). Steam-distillate of the leaves showed activity against *Mycobacterium tyberculosis*.

References

1. Sawyers CL, The New England Journal of Medicine, 340 (17): 1330-1340, 1999.
2. Vitale, C; Romagnani, C, et al., Proc. Natl. Acd. Sci. USA, 96 (26): 15091-15096, 1999.
3. Vitale, C et al., Proc. Natl. Acd. Sci, USA., 98 (10): 5764-5769,2001.
4. Sievers EL, Appelbaum, FR et al., Blood, 93: 3678-3684, 1999.
5. Ito H, Miyazaki T, Ono M and Sakurai H. Bioorg. Med. Chem. 6(7): 1051-1056, 1998.
6. Arion WJ *et. al*. Arch. Biochem. Biophys. 351(2): 279-285, 1998.
7. Conney AH *et.al*. Adv. Enzyme Regul. 31: 385-396, 1991.
8. Supriyatna G *et.al*. Phytomedicine, 7 (Suppl. II): 87, 2000.

**Objects of the invention**

The main objective of the invention is to provide a new use of the compound Chlorogenic acid isolated from the *Piper betel* leaf extract or from any other sources for the treatment of acute and chronic myeloid leukemia and lymphoid leukemia.

Another objective of the invention is to provide a new pharmaceutical composition comprising a carrier along with the compound Chlorogenic acid for the treatment of acute and chronic myeloid leukemia and lymphoid leukemia.

Yet another objective of the invention is to provide a process for the isolation of an active fraction from leaves or any other plant parts of *Piper betel* to treat AML, CML and lymphoid leukemia.

Yet another objective of the invention is to provide a simplified method of isolation of active components from all plant parts of *Piper betel* possessing biological activities relevant to the treatment of AML, CML and lymphoid leukemia.

Yet another objective of the invention is to provide a herbal product from leaves or any other plant parts of *Piper betel* for the treatment of AML, CML and lymphoid leukemia.

Yet another objective of the invention is to provide a herbal Chlorogenic acid purified from leaves of *Piper betel* for the treatment of AML, CML and lymphoid leukemia.

Yet another objective of the invention is to provide a process for the preparation of extract from leaves or any other plant parts of *Piper betel* for the treatment of AML, CML and lymphoid leukemia.

Yet another objective of the invention is to provide a simplified method of extract preparation from leaves or any other plant parts of *Piper betel* for the treatment of AML, CML and lymphoid leukemia.

Yet another objective of the invention is to provide a process for the preparation of Chlorogenic acid from leaves of *Piper betel* for the treatment of AML, CML and lymphoid leukemia.

Yet another objective of the invention is to provide herbal Chlorogenic acid purified from leaves or any other plant parts of *Piper betel* for the treatment of solid tumors including lymphomas.

Yet another objective of the invention is to provide new uses of Chlorogenic acid (isolated from any source or synthetically prepared) for the treatment of acute and chronic myeloid leukemia, lymphoid leukemia and solid tumors including lymphomas.

**Summary of the Invention**

Accordingly, the present invention provides a new use of the compound chlorogenic acid isolated from the piper betel leaf extract or from any other sources for the treatment of acute and chronic myeloid leukemia and lymphoid leukemia. In addition the present invention provides a pharmaceutical composition for acute and chronic myeloid leukemia in animals and humans, said composition comprising effective amount of chlorogenic acid (CA) and/or 3-o-p-Coumaryl quinic acid (PCQ), isolated from any plant parts of *Piper betel* or any other natural or synthetic source and pharmaceutical acceptable additives.

## Detailed Description of the Invention

In accordance, the present invention provides a pharmaceutical composition for acute and chronic myeloid leukemia in animals and humans, said composition comprising effective amount of chlorogenic acid (CA) and/or 3-o-p-Coumaryl quinic acid (PCQ), isolated from any plant parts of *Piper betel* or any other natural or synthetic source and pharmaceutical acceptable additives.

One embodiment of the invention, the additive is selected from a group consisting of nutrients such as proteins, carbohydrates, sugars, talc, magnesium stearate, cellulose, calcium carbonate, starch-gelatin paste and/or pharmaceutically acceptable carriers, excipient, diluents or solvents.

Another embodiment, ratio of CA and PCQ present in the composition ranging from 1:0 to 1:10, which is effective for the treatment of solid tumors including lymphomas.

Still another embodiment, the said composition is administered through oral, intravenous, intramuscular or subcutaneous routes.

Yet another embodiment, the composition is administered at a dose level ranging between 1 and 50 mg per kg body weight/day for at least for a period of four weeks.

Yet another embodiment, the composition is administered for a period ranging between four weeks and twelve weeks.

In yet another embodiment, the growth inhibition of leukemic cell lines $2X10^6$/ml/well of cell type K562 is up to 20% at a dose level of 25 $\mu$g/ml of CA.

In yet another embodiment, the growth inhibition of leukemic cell lines $2X10^6$/ml/well of cell type K562 is up to 4% at a dose level of 25 $\mu$g/ml of PCQ.

In yet another embodiment, the growth inhibition of leukemic cell lines $2X10^6$/ml/well of cell type K562 is up to 44% at a dose level containing 25 $\mu$g/ml each of CA and PCQ.

In yet another embodiment, the growth inhibition of leukemic cell lines $2X10^6$/ml/well of cell type U937 is up to 16.67 at a dose level of 25 $\mu$g/ml of CA.

In yet another embodiment, the growth inhibition of leukemic cell lines $2X10^6$/ml/well of cell type U937 is up to 2.08% at a dose level of 25 $\mu$g/ml of PCQ.

In yet another embodiment, the growth inhibition of leukemic cell lines $2X10^6$/ml/well of cell type U937 is up to 50% at a dose level containing 25 $\mu$g/ml each of CA and PCQ.

In yet another embodiment, the growth inhibition of leukemic cell lines $2X10^6$/ml/well of cell CML leukemic cells is up to 2.38 at a dose level of 25 $\mu$g/ml of CA.

In yet another embodiment, the growth inhibition of leukemic cell lines $2X10^6$/ml/well of CML leukemic cells is up to 2.38% at a dose level of 25 $\mu$g/ml of PCQ.

In yet another embodiment, the growth inhibition of leukemic cell lines $2X10^6$/ml/well of CML leukemic cells is up to 20.3% at a dose level containing 25 $\mu$g/ml each of CA and PCQ.

One more embodiment provides the use of composition for the treatment of acute and chronic myeloid leukemia (AML & CML) and lymphoid leukemia by administering a pharmaceutical composition comprising effective amount of chlorogenic acid (CA) and/or 3-o-p-Coumaryl quinic acid (PCQ), isolated from any plant parts of *Piper betel* or any other source and/or in combination with pharmaceutically acceptable additives.

The invention is described herein below with reference to examples, which are illustrative only and should not be construed to limit the scope of the present invention in any manner.

## Brief description of the accompanying drawings

Figure 1 represents HPLC of Fraction E showing the retention time (RE) of different peaks be divided into three zones, $Z_A$, $Z_B$ and $Z_C$.

Figure 1a. The peaks have been numbered ($Z_A$ had 4 major peaks whereas $Z_B$ had very small to moderate 10 peaks and $Z_C$ had two distinct peaks)

Figure 3 represents the structure of Chlorogenic acid (CA) or 3-Caffeoyl quinic acid.

Figure 4 represents photomicrographs of Leukemic cell lines, CML Patient's and normal donor's PBMC after treatment with CA.

**Example-1**

**Collection of plant material**

The leaves and all other plant parts of *Piper betel* were collected from the climber from different areas and West Bengal, India. A voucher specimen was deposited at the Deptt. of Medicinal Chemistry at the Indian Inst. of Chemical Biology, 4 Raja S.C. Mullick Road, Kolkata-700 032.

**Example 2: Isolation of compound Chlorogenic acid:**

4.7Kg of *Piper betel* leaves freshly collected, washed with distilled water and then cut into small pieces. Small pieces of leaves were gathered together, mixed with 1.0 litre of distilled water, and thoroughly homogenized in a mixture blender. The homogenate was passed through a fine cheesecloth to filter out the large particles and the filtrate was collected. The process was repeated 2-3 times to have maximum yield. The combined filtrate was then centrifuged, the aliquot, a clear solution, was collected and lyophilised to a semi-solid mass, which was about 110 gm.

Collected material was examined for biological activity i.e. destruction of leukemic cells. On observing its positive activity, purification was initiated. 10 gm of above-mentioned material was loaded on Sephadex LH-20 column and chromatographed with water, water-methanol (1:1) and methanol as eluent. Three different fractions thus obtained from three different solvent systems were separately checked for biological activity. The activity was located only on

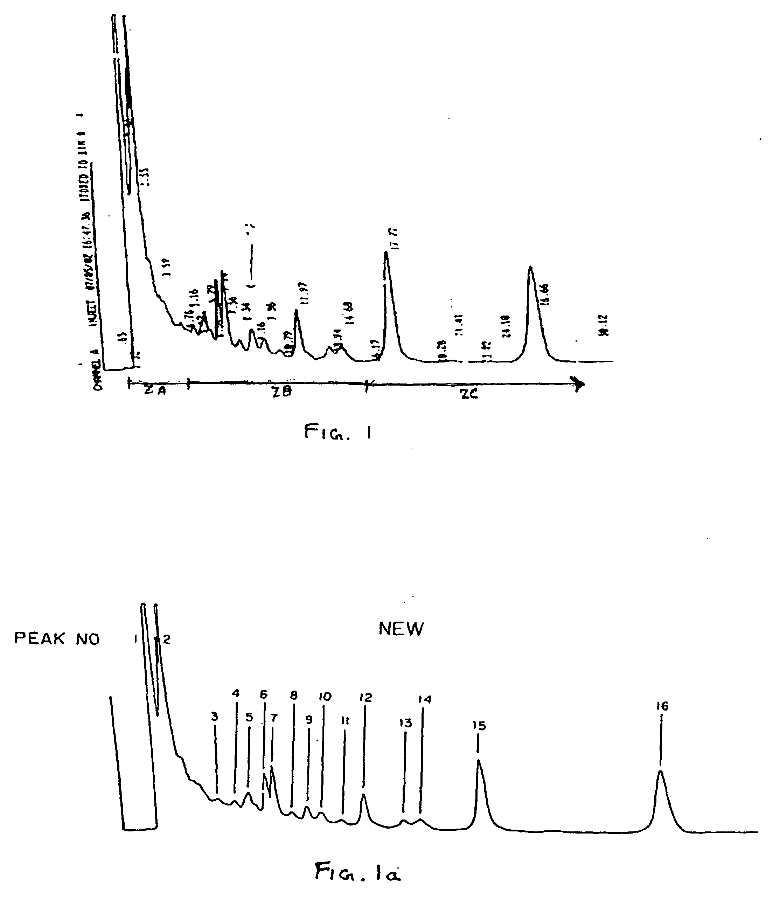

Fraction 2 i.e. Methanol-water (1:1) and termed as fraction E. Fraction E was then subjected to HPLC analysis using Intersil ODS-3 analytical column. The column was equilibrated with methanol-water-acetic acid (23:76:1), flow rate was maintained at 1.0 ml per min and peaks were identified at 280 nm. According to the retention time (RE) different peaks could be divided into three zones, $Z_A$, $Z_B$ and $Z_C$ (Fig. 1). $Z_A$ had 4 major peaks whereas $Z_B$ had very small to moderate 10 peaks and $Z_C$ had two distinct peaks. It was convenient to observe biological activity of three zones $A_A$, $Z_B$ and $Z_C$, which were separately tested on leukemia cells for destructive activity. All three showed biological activity.

It may be noted here that $Z_C$ which had two distinct peaks with retention time ($R_t$) 17.77 and 26.66 min. $R_t$ 17.77 min peak demonstrated considerable activity and therefore its structure was determined and filed for patent (299 /NF / 2002; dated-30.05.2002). The latter i.e. $R_t$ 26.66 min peak did not show any activity.

The Zone $Z_B$ contained 10 peaks (1 to 10) of varied sizes. Pooled 1-10 peaks fraction demonstrated leukemic cell killing activity. For this reason all different peaks were separately monitored for biological activity. Major peaks amongst them were 2,3,4 and 8 corresponding to $R_t$ 6.58, 7.14, 7.56 and 11.97 respectively and none of them exhibited activity. Hence minor peaks were separately examined by collecting repeated fractions for each peaks to increase the amount of active molecules present there. On searching for activity of these peaks i.e. 1,5,6,7,9 and 10 corresponding to $R_t$ 5.16, 8.34, 9.16, 9.86, 13.94 and 14.68 respectively. Except peak no 6 ($R_t$ 9.16 min) no other peaks had shown detectable activity. Peak 6 ($R_t$ 9.16 min) pooled fractions were lyophilized and reran through HPLC, the sharp single peak indicate its purity (Fig.2). This was then subjected to IR, NMR and Mass spectral analysis to determine the structure.

| IR $_{\gamma max}^{KBr}$ cm$^{-1}$ | 3355(OH), 1689(CO), 1637, 1604 1522, 1443, 1286, 1189, 1121, 1082 1039, 975 and 854 |
|---|---|
| $^1$H-NMR (CD$_3$OD) | 7.57(1H), 7.06 (1H), 6.96(1H), 6.79(1H) 6.27(1H), 5.34(1H), 4.17(1H), 3.73(1H) and 2.13 (4H) |
| $^{13}$C NMR (CD$_3$OD) | 176.00, 167.65, 148.56, 146.08, 145.79, 126.78, 121.98, 115.46, 114.24, 75.11, 72.45, 70.96, 70.26, 37.75 and 37.19 |
| FABMS m/Z | 355 (M$^+$+H) and 377 (M$^+$+Na) |

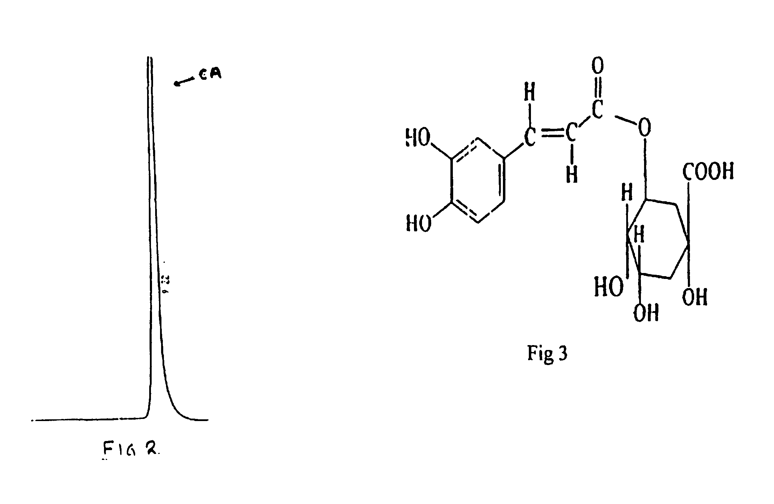

The structure thus determined shows it to be chlorogenic acid or 3-Caffeoyl quinic acid, m.p. 205° - 206° C [α]$_D$-33.25 (H$_2$O) (Fig.3). The chlorogenic acid is available in the market in pure form. This was bought and compared with the chlorogenic acid isolated from *Piper betle* leaves, which clearly confirmed that, the available material as chlorogenic acid.

Structure filed for patent earlier was 3-p-coumaryl quinic acid which showed CD33 + myeloid cells destruction but not CD33 - cells, and this is very mimilar to chlorogenic acid. Except the additional hydroxyl group at C-3 position of the aromatic ring in chlorogenic acid other structures are precisely similar. Therefore, the additional activity of chlorogenic acid over the 3-p-coumaryl quinic acid can be suggested to be due to the presence of hydroxyl group at C-3 position of aromatic ring. This specific difference has given broader and stronger activity to Chlorogenic acid in destroying both CD33 - and CD33 + cells and also lymphoid leukemic cells.

Zone $Z_A$ demonstrated bilogical activity. It contained 4 peaks, 1,2,3 and 4 corresponding to $R_t$ 0.65, 1.32, 1.8 and 2.55 respectively. Since separation of individual peak under $Z_A$ becomes difficult, location of peak wise activity could not be determined.

**Example 3:**

Preparation of peripheral blood mononuclear cells (PBMC) from myeloid leukemia patients.

Whole blood (10 ml each) was drawn from previously diagnosed myeloid leukemic patients, one patient was diagnosed as AML and the other one was diagnosed as CML. Mononuclear cells were separated by Ficoll/hypaque density gradient centrifugation.

**Example 4:**

Culture of Erythroleukemic cell line K562. This cell line was collected from American Type Culture Collection (ATCC), VA, USA. This cell line is CD33⁻. K562 cells were grown *in vitro* in Medium RPMI-1640 containing 10% heat inactivated fetal calf serum.

**Example 5:**

Culture of Promonocytic cell line U937. This cell line was obtained from American Type Culture Collection (ATCC), VA, USA, and grown *in vitro* as described for the cell line K562. This cell line is CD33⁺.

**Example 6**

Incubation of PBMC of myeloid leukemia patients with Chlorogenic acid (CA) *in vitro.*

PBMC ($1 \times 10^5$ - $2 \times 10^6$/ml) of myeloid leukemia patients were incubated with varied concentrations of CA for 48 hours at 37°C in 5% $CO_2$. Cells were then washed and counted for viability.

**Example 7**

Incubation of CD33⁺ cell line U937 with CA. U937 cells ($1 \times 10^5$ - $2 \times 10^6$/ml) were incubated with varying concentrations of CA for 48 hours at 37°C in 5% $CO_2$. Cells were then washed and counted for viability.

**Example 8**

Incubation of CD33⁻ cell line K562 with CA. K562 cells ($1 \times 10^5$ - $2 \times 10^6$/ml) were incubated with varying concentrations of CA for 48 hours at 37°C in 5% $CO_2$. Cells were then washed and counted for viability.

**Example 9**

Culture of T-Lymphoid cell line Molt-4. This cell line was obtained from American Type culture collection (ATCC), VA, USA and grown *in vitro* as described for the cell line K562.

**Example 10**

Preparation of peripheral blood mononuclear cells (PBMC) from normal individuals. Whole blood was collected and mononuclear cells were separated by Ficoll/Hypaque density gradient centrifugation.

**Example 11**

Analysis of cell cycle progression and apoptosis by Flow cytometry PBMC of a CML patient was cultured in RPMI-1640 medium supplemented with 10% heat-activated fetal bovine serum in the presence or absence of CA (100.0 µg/ml) for 48 hr. at 37°C in 5% $CO_2$. Cells were fixed with 40% ethanol treated with 500 µg/ml Rnase A, and then with 69 µM propidium iodide for analysis of DNA content by Flow Cytometry as described (Mitra et al., Molecular Medicine, 6:527-541, 2000).

**Results of Examples 6, 7 & 8:**

As shown in Table 1, PBMC of myeloid leukemia patients, CD33[+] Promonocytic cell line U937 and CD33[-] Erythroleukemic cell line K562 were destroyed by Chlorogenic acid.

**Results of Example 9 and 10:**

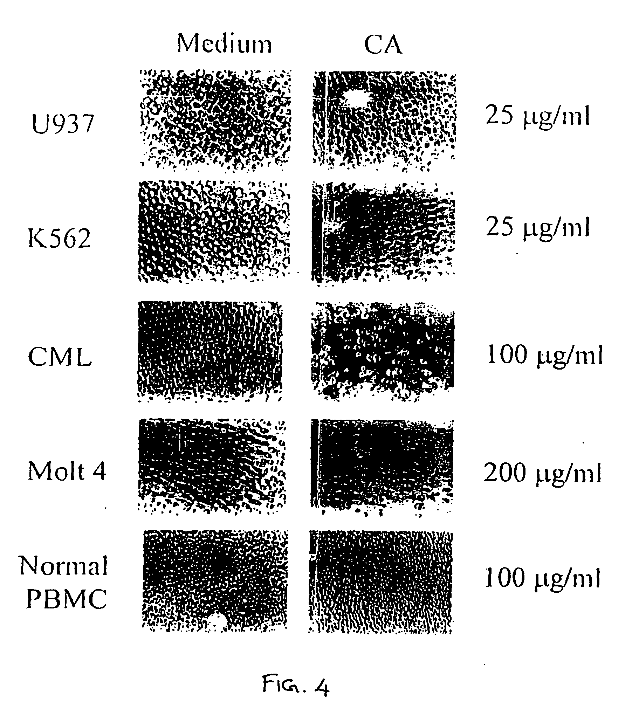

As shown Table 2, Erythroleukemia cell line K562, Pro-monocyte cell line U937 and CML patient's leukemic cells are destroyed by Chlorogenic acid T-lymphoid cell line Molt-4 requires higher dose. In contrast, PBMC of normal donors are virtually unaffected by CA. Photomicrographs of U937, K562, CML Patient's PBMC, Molt-4 and normal PBMC after culturing for 48 hr. in the presence or absence of CA are shown in Fig. 4. We claimed earlier that 3-o-p-Coumaryl quinic acid (PCQ) has anti myeloid leukemia acitivity (299/NF/2002; 30.05.2002). Here we show that combination of chlorogenic acid (CA) and 3-o-p-coumaryl quinic acid at a ratio of 1:1 is more effective than CA or PCQ alone in destroying Leukemic cell lines or Myeloid Leukemia patient's Lekemic cells but have no effects on normal donor's PBMC (Table 3).

**Results of Example 11**

Cell-cycle analysis demonstrate that after two days of culture, chlorogenic acid at a concentration of 100.0 µg/ml caused apoptosis in CML patients Leukemic cells *in vitro* because of their accumulation in S, $G_2$ or M phase compared to the cells cultured in medium alone (Table 4).

Table-I:

| Chlorogenic acid destroys patients' leukemic cells and leukemia cell lines *in vitro*. | | | | |
|---|---|---|---|---|
| **Compound** | **Percent inhibition of cell growth after 48 hr of incubation** | | | |
| | AML patient's PBMC* | CML patient's PBMC | Promonocytic cell line (U937) | Erythroleukemic cell line (K562) |
| Medium alone | 0.0 | 0.0 | 0.0 | 0.0 |
| **Chlorogenic acid (CA)** | | | | |
| 10 µg/ml | ND | ND | 25.0 | 46.6 |
| 15 µg/ml | ND | ND | 43.7 | 93.3 |
| 20 µg/ml | 9.5 | 5.8 | 90.6 | 100.0 |
| 50 µg/ml | 16.6 | 10.3 | - | - |
| 100 µg/ml | 73.8 | 22.5 | - | - |
| 500 µg/ml | 85.7 | 32.3 | - | - |
| Note: 1. PBMC*, Peripheral blood mononuclear cells. **2. The number of cells used was 1x10$^5$/ml/well** | | | | |

Table 2:

| Growth Inhibition of Leukemic Cell Lines and CML Patient's Leukemic Cells by CA *in vitro*. | | | | |
|---|---|---|---|---|
| Cells | Incubated with (µg/ml) | Percentage inhibition of Cell Growth | | |
| | | 1 day | 2 days | 3 days |
| K562 | Medium | 0.00 | 0.00 | 0.00 |
| | CA (10.0) | 8.00 | 48.14 | 85.18 |
| | CA (25.0) | 20.00 | 62.96 | 92.59 |
| | CA (50.0) | 28.00 | 77.78 | 100.0 |
| | CA (100.0) | 84.00 | 98.14 | 100.0 |

Table 2: (continued)

| Growth Inhibition of Leukemic Cell Lines and CML Patient's Leukemic Cells by CA *in vitro.* | | | | |
|---|---|---|---|---|
| Cells | Incubated with (µg/ml) | Percentage inhibition of Cell Growth | | |
| | | 1 day | 2 days | 3 days |
| U937 | Medium | 0.00 | 0.00 | 0.00 |
| | CA (10.0) | 8.33 | 44.0 | 57.14 |
| | CA(25.0) | 16.66 | 52.00 | 71.42 |
| | CA (50.0 | 25.00 | 64.00 | 89.28 |
| | CA (100.0) | 45.83 | 96.00 | 100.0 |
| Molt 4 (T lymphoid Cell line) | Medium | 0.00 | 0.00 | 0.00 |
| | CA (50.0) | 3.57 | 6.67 | 12.5 |
| | CA (100.0) | 6.89 | 14.67 | 18.75 |
| | CA (200.0) | 13.79 | 46.67 | 62.5 |
| CML Patient's PBMC | Medium | 0.00 | 4.76 | 14.28 |
| | CA (50.0) | 4.76 | 22.22 | 30.0 |
| | CA (100.0 | 9.52 | 45.0 | 55.56 |
| Normal PBMC | Medium | 0.00 | 3.33 | 3.33 |
| | CA (50.0) | 6.66 | 10.34 | 15.2 |
| | CA (100.0) | 6.66 | 17.24 | 19.5 |

Of note, the number of cells used was $1 \times 10^5$ per ml per well.

Table 3.

| Combination of Chlorogenic acid (CA) and 3-o-p-Coumaryl Quinic acid (PCQ) is more effective in destroying Leukemic cell lines and AML and CML patient's Leukemic cells *in vitro* | | |
|---|---|---|
| Cells | Incubated with | % Inhibition of cell growth (After 3 days of incubation) |
| K562 Erythroleukemic cell line | Media | 0.00 |
| | CA (25.0 µg/ml) | 20.00 |
| | CA (50.0 µg/ml) | 28.00 |
| | PCQ (25.0 µg/ml) | 4.00 |
| | PCQ (50.0 µg/ml) | 8.00 |
| | CA (25.0µg/ml)+ PCQ (25.0 µg/ml) | 44.00 |
| U937 Promonocyte cell line | Media | 0.00 |
| | CA (25.0 µg/ml) | 16.67 |
| | CA (50.0 µg/ml)' | 25.00 |
| | PCQ (25.0 µg/ml) | 2.08 |
| | PCQ (50.0 µg/ml) | 4.17 |
| | CA (25.0 µg/ml) + PCQ (25.0 µg/ml) | 50.00 |

Table 3.   (continued)

| Combination of Chlorogenic acid (CA) and 3-o-p-Coumaryl Quinic acid (PCQ) is more effective in destroying Leukemic cell lines and AML and CML patient's Leukemic cells *in vitro* | | |
| --- | --- | --- |
| Cells | Incubated with | % Inhibition of cell growth (After 3 days of incubation) |
| CML Patient's Leukemic cells | Media | 0.00 |
| | CA (25.0 µg/ml) | 2.38 |
| | CA (50.0 µg/ml)' | 4.67 |
| | PCQ (25.0 µg/ml) | 2.38 |
| | PCQ (50.0 µg/ml) | 4.76 |
| | CA (25.0 µg/ml) + PCQ (25.0 µg/ml) | 20.3 |
| Normal individual's PBMC | Media | 0.00 |
| | CA (25.0 µg/ml) | 1.03 |
| | CA (50.0 µg/ml)' | 1.03 |
| | PCQ (25.0 µg/ml) | 0.00 |
| | PCQ (50.0 µg/ml) | 1.67 |
| | CA (25.0 µg/ml) + PCQ (25.0 µg/ml) | 1.03 |

The number of cells used was $2 \times 10^5$ per ml per well. The percentage of killing of cells by these compounds is dependent on the number of cells present per ml.

Table 4

| The percentage of cells in different phases of cell cycle after treatment with CA for 2 days. | | | | |
| --- | --- | --- | --- | --- |
| Type of cells | Incubated with | Sub G0/G1 (apoptotic 2n DNA) | G0/G1 (2n DNA) | 2+G2+M (>2n DNA) |
| | Media | 4.65 | 51.56 | 43.87 |
| CML Patient's Leukemic Cells | CA (100.0 µg/ml) | 10.64 | 33.13 | 56.35 |

**Claims**

1. A synergistic pharmaceutical composition for acute and chronic myeloid leukemia in animals and humans, said composition comprising an effective amount of chlorogenic acid (CA) and 3-o-p-Coumaryl quinic acid (PCQ), isolated from any plant parts of *Piper betel* or any other natural or synthetic source, and pharmaceutical acceptable additives.

2. The composition as claimed in claim 1 wherein, the additive is selected from a group consisting of nutrients such as proteins, carbohydrates, sugars, talc, magnesium stearate, cellulose, calcium carbonate, starch-gelatin paste and/or pharmaceutically acceptable carriers, excipient, diluents or solvents.

3. The composition as claimed in claim 1 wherein ratio of CA and PCQ present in the composition ranging from 1:0 to 1:10, which is effective for the treatment of solid tumors including lymphomas.

4. The composition as claimed in claim 1, the said composition is to be administered through oral, intravenous, intramuscular or subcutaneous routes.

5. The composition as claimed in claim 1 to be administered at a dose level ranging between 1 and 50 mg per kg body weight/day for at least for a period of four weeks.

6. The composition as claimed in claim 1, which is to be administered for a period ranging between four weeks and twelve weeks.

7. The composition as claimed in claim 1 wherein, the growth inhibition of leukemic cell lines $2X10^6$/ml/well of cell type K562 is up to 20% at a dose level of 25 µg/ml of CA.

8. The composition as claimed in claim 1, wherein the growth inhibition of leukemic cell lines $2X10^6$/ml/well of cell type K562 is up to 4% at a dose level of 25 µg/ml of PCQ.

9. The composition as claimed in claim 1, wherein the growth inhibition of leukemic cell lines $2X10^6$/ml/well of cell type K562 is up to 44% at a dose level containing 25 µg/ml each of CA and PCQ.

10. The composition as claimed in claim 1 wherein, the growth inhibition of leukemic cell lines $2X10^6$/ml/well of cell type U937 is up to 16.67 at a dose level of 25 µg/ml of CA.

11. The composition as claimed in claim 1, wherein the growth inhibition of leukemic cell lines $2X10^6$/ml/well of cell type U937 is up to 2.08% at a dose level of 25 µg/ml of PCQ.

12. The composition as claimed in claim 1, wherein the growth inhibition of leukemic cell lines $2X10^6$/ml/well of cell type U937 is up to 50% at a dose level containing 25 µg/ml each of CA and PCQ.

13. The composition as claimed in claim 1 wherein, the growth inhibition of leukemic cell lines $2X10^6$/ml/well of cell CML leukemic cells is up to 2.38 at a dose level of 25 µg/ml of CA.

14. The composition as claimed in claim 1, wherein the growth inhibition of leukemic cell lines $2X10^6$/ml/well of CML leukemic cells is up to 2.38% at a dose level of 25 µg/ml of PCQ.

15. The composition as claimed in claim 1, wherein the growth inhibition of leukemic cell lines $2X10^6$/ml/well of CML leukemic cells is up to 20.3% at a

16. Use of composition as claimed in claim 1, for the preparation of a medicament for the treatment of acute and chronic myeloid leukemia (AML & CML) and lymphoid leukemia by using a pharmaceutical composition comprising effective amount of chlorogenic acid (CA) and/or 3-o-p-Coumaryl quinic acid (PCQ), isolated from any plant parts of *Piper betel* or any other source and/or in combination with pharmaceutically acceptable additives.

17. A use as claimed in claim 16 wherein, CA and PCQ both are isolated from any plant parts of *Piper betel* or are synthetically prepared.

18. A use as claimed in claim 16 wherein the subject is selected from a mammal preferably a human being.

19. A use as claimed in claim 16 wherein, the additive is selected from a group consisting of nutrients such as proteins, carbohydrates, sugars, talc, magnesium stearate, cellulose, calcium carbonate, starch-gelatin paste and/or pharmaceutically acceptable carriers, excipient, diluents or solvents.

20. A use as claimed in claim 16 wherein ratio of CA and PCQ present in the composition ranging from 1:0 to 1:10, is effective for the treatment of solid tumors including lymphomas.

21. A use as claimed in claim 16 wherein, the said composition is to be administered through oral, intravenous, intramuscular or subcutaneous routes.

22. A use as claimed in claim 16 is to be administered at dose levels between 1 to 50 mg per kg body weight at least once in a day for a period of four weeks.

23. A use as claimed in claim 16, which is to be administered for a period of four weeks and up to three months.

24. A use as claimed in claim 16 wherein, the growth inhibition of leukemic cell lines $2X10^6$/ml/well of cell type K562 is up to 20% at a dose level of 25 µg/ml of CA.

**25.** A use as claimed in claim 16, wherein the growth inhibition of leukemic cell lines $2X10^6$/ml/well of cell type K562 is up to 4% at a dose level of 25 µg/ml of PCQ.

**26.** A use as claimed in claim 16, wherein the growth inhibition of leukemic cell lines $2X10^6$/ml/well of cell type K562 is up to 44% at a dose level containing 25 µg/ml each of CA and PCQ.

**27.** A use as claimed in claim 16, wherein, the growth inhibition of leukemic cell lines $2X10^6$/ml/well of cell type U937 is up to 16.67 at a dose level of 25 µg/ml of CA.

**28.** A use as claimed in claim 16, wherein the growth inhibition of leukemic cell lines $2X10^6$/ml/well of cell type U937 is up to 2.08% at a dose level of 25 µg/ml of PCQ.

**29.** A use as claimed in claim 16, wherein the growth inhibition of leukemic cell lines $2X10^6$/ml/well of cell type U937 is up to 50% at a dose level containing 25 µg/ml each of CA and PCQ.

**30.** A use as claimed in claim 16, wherein the growth inhibition of leukemic cell lines $2X10^6$/ml/well of cell CML leukemic cells is up to 2.38 at a dose level of 25 µg/ml of CA.

**31.** A use as claimed in claim 16, wherein the growth inhibition of leukemic cell lines $2X10^6$/ml/well of CML leukemic cells is up to 2.38% at a dose level of 25 µg/ml of PCQ.

**32.** A use as claimed in claim 17, wherein the growth inhibition of leukemic cell lines $2X10^6$/ml/well of CML leukemic cells is up to 20.3% at a dose level containing 25 µg/ml each of CA and PCQ.

**Patentansprüche**

**1.** Synergistische pharmazeutische Zusammensetzung für akute und chronische myeloische Leukämie bei Tieren und Menschen, wobei die Zusammensetzung eine wirksame Menge von Chlorogensäure (CA) und 3-o-p-Cumarylchinasäure (PCQ), die aus beliebigen Pflanzenteilen von *Piper betel* oder einer beliebigen anderen natürlichen oder synthetischen Quelle isoliert wurden, und pharmazeutisch akzeptable Additive umfasst.

**2.** Zusammensetzung gemäß Anspruch 1, wobei das Additiv aus einer Gruppe, die aus Nährstoffen, wie Proteinen, Kohlenhydraten, Zuckern, Talkum, Magnesiumstearat, Cellulose, Calciumcarbonat, Stärke-Gelatinepaste, und/oder pharmazeutisch akzeptablen Trägern, Streckmitteln, Verdünnungsmitteln oder Lösemitteln besteht, ausgewählt ist.

**3.** Zusammensetzung gemäß Anspruch 1, wobei das Verhältnis der in der Zusammensetzung vorhandenen CA und PCQ im Bereich von 1:0 bis 1:10 liegt, was zur Behandlung von soliden Tumoren einschließlich Lymphomen wirksam ist.

**4.** Zusammensetzung gemäß Anspruch 1, wobei die Zusammensetzung auf oralem, intravenösem, intramuskulärem oder subkutanem Weg zu verabreichen ist.

**5.** Zusammensetzung gemäß Anspruch 1, die in einer Dosishöhe im Bereich zwischen 1 und 50 mg pro kg Körpergewicht/Tag während mindestens eines Zeitraums von 4 Wochen zu verabreichen ist.

**6.** Zusammensetzung gemäß Anspruch 1, die während eines Zeitraums im Bereich zwischen 4 Wochen und 12 Wochen zu verabreichen ist.

**7.** Zusammensetzung gemäß Anspruch 1, wobei die Wachstumshemmung von Leukämiezelllinien bei $2 \times 10^6$/ml/ Vertiefung des Zelltyps K562 bis zu 20 % bei einer Dosismenge von 25 µg/ml CA beträgt.

**8.** Zusammensetzung gemäß Anspruch 1, wobei die Wachstumshemmung von Leukämiezelllinien bei $2 \times 10^6$/ml/ Vertiefung des Zelltyps K562 bis zu 4 % bei einer Dosismenge von 25 µg/ml PCQ beträgt.

**9.** Zusammensetzung gemäß Anspruch 1, wobei die Wachstumshemmung von Leukämiezelllinien bei $2 \times 10^6$/ml/ Vertiefung des Zelltyps K562 bis zu 44 % bei einer Dosismenge, die 25 µg/ml von jeweils CA und PCQ enthält, beträgt.

10. Zusammensetzung gemäß Anspruch 1, wobei die Wachstumshemmung von Leukämiezelllinien bei 2 x $10^6$/ml/ Vertiefung des Zelltyps U937 bis zu 16,67 % bei einer Dosismenge von 25 µg/ml CA beträgt.

11. Zusammensetzung gemäß Anspruch 1, wobei die Wachstumshemmung von Leukämiezelllinien bei 2 x $10^6$/ml/ Vertiefung des Zelltyps U937 bis zu 2,08 % bei einer Dosismenge von 25 µg/ml PCQ beträgt.

12. Zusammensetzung gemäß Anspruch 1, wobei die Wachstumshemmung von Leukämiezelllinien bei 2 x $10^6$/ml/ Vertiefung des Zelltyps U937 bis zu 50 % bei einer Dosismenge, die 25 µg/ml von jeweils CA und PCQ enthält, beträgt.

13. Zusammensetzung gemäß Anspruch 1, wobei die Wachstumshemmung von Leukämiezelllinien bei 2 x $10^6$/ml/ Vertiefung des Zelltyps CML-Leukämiezellen bis zu 2,38 % bei einer Dosismenge von 25 µg/ml CA beträgt.

14. Zusammensetzung gemäß Anspruch 1, wobei die Wachstumshemmung von Leukämiezelllinien bei 2 x $10^6$/ml/ Vertiefung des Zelltyps CML-Leukämiezellen bis zu 2,38 % bei einer Dosismenge von 25 µg/ml PCQ beträgt.

15. Zusammensetzung gemäß Anspruch 1, wobei die Wachstumshemmung von Leukämiezelllinien bei 2 x $10^6$/ml/ Vertiefung des Zelltyps CML-Leukämiezellen bis zu 20,3 % bei einer Dosismenge, die 25 µg/ml von jeweils CA und PCQ enthält, beträgt.

16. Verwendung einer Zusammensetzung gemäß Anspruch 1 zur Herstellung eines Medikaments zur Behandlung von akuter und chronischer myeloischer Leukämie (AML & CML) und lymphatischer/lympocytischer Leukämie durch Verwendung einer pharmazeutischen Zusammensetzung, die eine wirksame Menge von Chlorogensäure (CA) und/oder 3-o-p-Cumarylchinasäure (PCQ), die aus beliebigen Pflanzenteilen von *Piper betel* oder einer beliebigen anderen Quelle isoliert wurden, und/oder in Kombination mit pharmazeutisch akzeptablen Additiven umfasst.

17. Verwendung gemäß Anspruch 16, wobei CA und PCQ beide aus beliebigen Pflanzenteilen von *Piper betel* isoliert oder synthetisch hergestellt sind.

18. Verwendung gemäß Anspruch 16, wobei das Subjekt aus einem Säuger ausgewählt und vorzugsweise ein Mensch ist.

19. Verwendung gemäß Anspruch 16, wobei das Additiv aus einer Gruppe, die aus Nährstoffen, wie Proteinen, Kohlenhydraten, Zuckern, Talkum, Magnesiumstearat, Cellulose, Calciumcarbonat, Stärke-Gelatinepaste, und/oder pharmazeutisch akzeptablen Trägern, Streckmitteln, Verdünnungsmitteln oder Lösemitteln besteht, ausgewählt ist.

20. Verwendung gemäß Anspruch 16, wobei das Verhältnis der in der Zusammensetzung vorhandenen CA und PCQ, das im Bereich von 1:0 bis 1:10 liegt, zur Behandlung solider Tumore einschließlich Lymphomen wirksam ist.

21. Verwendung gemäß Anspruch 16, wobei die Zusammensetzung auf oralem, intravenösem, intramuskulärem oder subkutanem Weg zu verabreichen ist.

22. Verwendung gemäß Anspruch 16, wobei die Zusammensetzung in Dosismengen zwischen 1 und 50 mg pro kg Körpergewicht mindestens einmal täglich über einen Zeitraum von 4 Wochen zu verabreichen ist.

23. Verwendung gemäß Anspruch 16, wobei die Zusammensetzung über einen Zeitraum von 4 Wochen und bis zu 3 Monaten zu verabreichen ist.

24. Verwendung gemäß Anspruch 16, wobei die Wachstumshemmung von Leukämiezelllinien bei 2 x $10^6$/ml/Vertiefung des Zelltyps K562 bis zu 20 % bei einer Dosismenge von 25 µg/ml CA beträgt.

25. Verwendung gemäß Anspruch 16, wobei die Wachstumshemmung von Leukämiezelllinien bei 2 x $10^6$/ml/Vertiefung des Zelltyps K562 bis zu 4 % bei einer Dosismenge von 25 µg/ml PCQ beträgt.

**26.** Verwendung gemäß Anspruch 16, wobei die Wachstumshemmung von Leukämiezelllinien bei 2 x $10^6$/ml/Vertiefung des Zelltyps K562 bis zu 44 % bei einer Dosismenge, die 25 µg/ml von jeweils CA und PCQ enthält, beträgt-

**27.** Verwendung gemäß Anspruch 16, wobei die Wachstumshemmung von Leukämiezelllinien bei 2 x $10^6$/ml/Vertiefung des Zelltyps U937 bis zu 16,67 % bei einer Dosismenge von 25 µg/ml CA beträgt.

**28.** Verwendung gemäß Anspruch 16, wobei die Wachstumshemmung von Leukämiezelllinien bei 2 x $10^6$/ml/Vertiefung des Zelltyps U937 bis zu 2,08 % bei einer Dosismenge von 25 µg/ml PCQ beträgt.

**29.** Verwendung gemäß Anspruch 16, wobei die Wachstumshemmung von Leukämiezelllinien bei 2 x $10^6$/ml/Vertiefung des Zelltyps U937 bis zu 50 % bei einer Dosismenge, die 25 µg/ml von jeweils CA und PCQ enthält, beträgt.

**30.** Verwendung gemäß Anspruch 16, wobei die Wachstumshemmung von Leukämiezelllinien bei 2 x $10^6$/ml/Vertiefung des Zelltyps CML-Leukämiezellen bis zu 2,38 % bei einer Dosismenge von 25 µg/ml CA beträgt.

**31.** Verwendung gemäß Anspruch 16, wobei die Wachstumshemmung von Leukämiezelllinien bei 2 x $10^6$/ml/Vertiefung des Zelltyps CML-Leukämiezellen bis zu 2,38 % bei einer Dosismenge von 25 µg/ml PCQ beträgt.

**32.** Verwendung gemäß Anspruch 17, wobei die Wachstumshemmung von Leukämiezelllinien bei 2 x $10^6$/ml/Vertiefung des Zelltyps CML-Leukämiezellen bis zu 20,3 % bei einer Dosismenge, die 25 µg/ml von jeweils CA und PCQ enthält, beträgt.

**Revendications**

**1.** Composition pharmaceutique synergique pour la leucémie myéloïde aiguë et chronique chez les animaux et les humains, ladite composition comprenant une quantité efficace d'acide chlorogénique (AC) et d'acide 3-o-p-coumaryl-quinique (PCQ), isolés à partir d'une quelconque des parties de la plante *Piper betel* ou d'une quelconque autre source naturelle ou synthétique, et des additifs acceptables sur le plan pharmaceutique.

**2.** Composition comme revendiquée selon la revendication 1, dans laquelle l'additif est choisi dans un groupe constitué de nutriments tels que les protéines, les hydrates de carbones, les sucres, le talc, le stéarate de magnésium, la cellulose, le carbonate de calcium, la pâte amidon/gélatine et/ou de véhicules, excipient, diluants ou solvants acceptables sur le plan pharmaceutique

**3.** Composition comme revendiquée selon la revendication 1, dans laquelle le rapport de AC sur PCQ présent dans la composition allant de 1 :0 à 1 :10, est efficace pour le traitement des tumeurs solides incluant les lymphomes.

**4.** Composition comme revendiquée selon la revendication 1, ladite composition devant être administrée par voie orale, intraveineuse, intramusculaire ou sous-cutanée.

**5.** Composition comme revendiquée selon la revendication 1 devant être administrée à un niveau de dose se trouvant entre 1 et 50 mg par kg de poids corporel et par jour pour au moins une période de quatre semaines.

**6.** Composition comme revendiquée selon la revendication 1 devant être administrée pendant une période allant de quatre semaines à douze semaines.

**7.** Composition comme revendiquée selon la revendication 1, dans laquelle l'inhibition de croissance des lignées cellulaires leucémiques de 2x$10^6$/mL/puits cellules du type K562 est jusqu'à 20 % à un niveau de dose de 25 µg/mL de AC.

**8.** Composition comme revendiquée selon la revendication 1, dans laquelle l'inhibition de croissance des lignées cellulaires leucémiques de 2x$10^6$/mL/puits cellules du type K562 est jusqu'à 4 % à un niveau de dose de 25 µg/mL de PCQ.

**9.** Composition comme revendiquée selon la revendication 1, dans laquelle l'inhibition de croissance des lignées cellulaires leucémiques de 2x$10^6$/mL/puits cellules du type K562 est jusqu'à 44 % à un niveau de dose contenant 25 µg/mL chacun de AC et de PCQ.

**10.** Composition comme revendiquée selon la revendication 1, dans laquelle l'inhibition de croissance des lignées cellulaires leucémiques de $2\times10^6$/mL/puits cellules du type U937 est jusqu'à 16,67% à un niveau de dose de 25 µg/mL de AC.

**11.** Composition comme revendiquée selon la revendication 1, dans laquelle l'inhibition de croissance des lignées cellulaires leucémiques de $2\times10^6$/mL/puits cellules du type U937 est jusqu'à 2,08 % à un niveau de dose de 25 µg/mL de PCQ.

**12.** Composition comme revendiquée selon la revendication 1, dans laquelle l'inhibition de croissance des lignées cellulaires leucémiques de $2\times10^6$/mL/puits cellules du type U937 est jusqu'à 50 % à un niveau de dose contenant 25 µg/mL chacun de AC et de PCQ.

**13.** Composition comme revendiquée selon la revendication 1, dans laquelle l'inhibition de croissance des lignées cellulaires leucémiques de $2\times10^6$/mL/puits cellules leucémiques CML est jusqu'à 2,38 % à un niveau de dose de 25 µg/mL de AC.

**14.** Composition comme revendiquée selon la revendication 1, dans laquelle l'inhibition de croissance des lignées cellulaires leucémiques de $2\times10^6$/mL/puits cellules leucémiques CML est jusqu'à 2,38 % à un niveau de dose de 25 µg/mL de PCQ.

**15.** Composition comme revendiquée selon la revendication 1, dans laquelle l'inhibition de croissance des lignées cellulaires leucémiques de $2\times10^6$/mL/puits cellules leucémiques CML est jusqu'à 20,3 % à un niveau de dose contenant 25 µg/mL chacun de AC et de PCQ.

**16.** Utilisation de la composition comme revendiquée selon la revendication 1, pour la préparation d'un médicament destiné au traitement de la leucémie myéloïde aiguë et chronique (LMA et LMC) et de la leucémie lymphoïde en utilisant une composition pharmaceutique comprenant une quantité efficace d'acide chlorogénique (AC) et/ou d'acide 3-o-p-Coumaryl-quinique (PCQ), isolés à partir d'une quelconque des parties de la plante *Piper betel* ou d'une quelconque autre source et/ou en combinaison avec des additifs acceptables sur le plan pharmaceutique.

**17.** Utilisation comme revendiquée selon la revendication 16, dans laquelle à la fois AC et PCQ sont isolés à partir d'une quelconque des parties de la plante *Piper betel* ou sont préparés par synthèse.

**18.** Utilisation comme revendiquée selon la revendication 16, dans laquelle le sujet est choisi parmi les mammifères, préférablement un être humain.

**19.** Utilisation comme revendiquée selon la revendication 16, dans laquelle l'additif est choisi dans un groupe constitué de nutriments tels que les protéines, les hydrates de carbones, les sucres, le talc, le stéarate de magnésium, la cellulose, le carbonate de calcium, la pâte amidon/gélatine et/ou de véhicules, excipient, diluants ou solvants acceptables sur le plan pharmaceutique.

**20.** Utilisation comme revendiquée selon la revendication 16, dans laquelle le rapport de AC sur PCQ présent dans la composition allant de 1 :0 à 1 :10, est efficace pour le traitement des tumeurs solides incluant les lymphomes.

**21.** Utilisation comme revendiquée selon la revendication 16, dans laquelle ladite composition doit être administrée par voie orale, intraveineuse, intramusculaire ou sous-cutanée.

**22.** Utilisation comme revendiquée selon la revendication 16 devant être administrée à un niveau de dose se trouvant entre 1 et 50 mg par kg de poids corporel et par jour au moins une fois par jour pendant une période de quatre semaines.

**23.** Utilisation comme revendiquée selon la revendication 16 devant être administrée pendant une période allant de quatre semaines jusqu'à trois mois.

**24.** Utilisation comme revendiquée selon la revendication 16, dans laquelle l'inhibition de croissance des lignées cellulaires leucémiques de $2\times10^6$/mL/puits cellules du type K562 est jusqu'à 20 % à un niveau de dose de 25 µg/mL de AC.

**25.** Utilisation comme revendiquée selon la revendication 16, dans laquelle l'inhibition de croissance des lignées cellulaires leucémiques de 2x10$^6$/mL/puits cellules du type K562 est jusqu'à 4 % à un niveau de dose de 25 µg/mL de PCQ.

**26.** Utilisation comme revendiquée selon la revendication 16, dans laquelle l'inhibition de croissance des lignées cellulaires leucémiques de 2x10$^6$/mL/puits cellules du type K562 est jusqu'à 44 % à un niveau de dose contenant 25 µg/mL chacun de AC et de PCQ.

**27.** Utilisation comme revendiquée selon la revendication 16, dans laquelle l'inhibition de croissance des lignées cellulaires leucémiques de 2x10$^6$/mL/puits cellules du type U937 est jusqu'à 16,67% à un niveau de dose de 25 µg/mL de AC.

**28.** Utilisation comme revendiquée selon la revendication 16, dans laquelle l'inhibition de croissance des lignées cellulaires leucémiques de 2x10$^6$/mL/puits cellules du type U937 est jusqu'à 2,08 % à un niveau de dose de 25 µg/mL de PCQ.

**29.** Utilisation comme revendiquée selon la revendication 16, dans laquelle l'inhibition de croissance des lignées cellulaires leucémiques de 2x10$^6$/mL/puits cellules du type U937 est jusqu'à 50 % à un niveau de dose contenant 25 µg/mL chacun de AC et de PCQ.

**30.** Utilisation comme revendiquée selon la revendication 16, dans laquelle l'inhibition de croissance des lignées cellulaires leucémiques de 2x10$^6$/mL/puits cellules leucémiques CML est jusqu'à 2,38 % à un niveau de dose de 25 µg/mL de AC.

**31.** Utilisation comme revendiquée selon la revendication 16, dans laquelle l'inhibition de croissance des lignées cellulaires leucémiques de 2x10$^6$/mL/puits cellules leucémiques CML est jusqu'à 2,38 % à un niveau de dose de 25 µg/mL de PCQ.

**32.** Utilisation comme revendiquée selon la revendication 16, dans laquelle l'inhibition de croissance des lignées cellulaires leucémiques de 2x10$^6$/mL/puits cellules leucémiques CML est jusqu'à 20,3 % à un niveau de dose contenant 25 µg/mL chacun de AC et de PCQ.

FIG. 1

FIG. 1a

Fig 3

FIG 2

FIG. 4